# EUROPEAN PATENT APPLICATION

(11) **EP 2 446 880 A1**
(43) Date of publication of application: **02.05.2012**
(21) Application number: 12151732.0
(22) Date of filing: 06.10.2008
(51) Int. Cl.: A61K 9/16, A61K 9/28

(54) **Film-coated tablets containing drospirenone as active agent and a method for the preparation thereof**

(30) Priority: 20.12.2007 HU 0700826
(62) Divisional of application: 08462008.7
(71) Applicant: Richter Gedeon NYRT, 1103 Budapest (HU)
(72) Inventor: Bódis, Attila, 1103 Budapest (HU); Greiner, István, 1103 Budapest (HU); Nagy Kasza, Jonathán Mihály, 1103 Budapest (HU)
(74) Representative: HOFFMANN EITLE

(57) **Abstract**

The present invention relates to a film-coated tablet containing drospirenone active agent, comprising a tablet core coated with a film-forming polymer layer with improved resistance against atmospheric humidity. The tablet core is obtainable by a process which comprises dissolving drospirenone in ethanol, then dripping the solution obtained onto the surface of the fluid bed in a fluidizing equipment without spraying pressure while controlling the temperature of the product obtained and removing the solvent molecules from the wet material by a hot airflow.

## Description

### Field of the invention

The invention relates to drospirenone containing film-coated tablet with improved resistance against the environmental influences, especially against atmospheric humidity. The invention further relates to a method for the preparation film-coated tablet cores whereby applying the active agent to the core may be accomplished with increased safety.

### Background of the invention

Contraceptives, containing combinations of a gestagen and an estrogen component has been used since some decades. The drospirenone (chemical name: 6β, 7β; 15β, 16β-dimethylene-3-oxo-17α-pregn-4-en-21,17-carbolactone) is known from DE 2652761 patent specification in which its use as a diuretic compound is disclosed. In DE 3022337 patent specification the progestin-like activity of the compound and its consequent utility as contraceptive agent was described. It is further described in DE 3022337 specification that drospirenone may also be administered together with ethynylestradiol.

In case of hormone-containing compositions generally two kinds of wet granulating methods are used in the pharmaceutical industry. One of them is the Foucault current granulation method, which recently can be combined with microwave vacuum drying. When compared to the fluidized bed drying method disadvantages of the Foucault current single pot method are the long operating time during the drying procedure, and the inhomogeneous heat distribution which may occurred incidentally and may result in a "hot spot" phenomenon. The above two effects may influence the quality of the product adversely. Another method is the fluidized bed drying method, which cannot be considered as a single pot technology since during the procedure the wet mass must be transferred into a drying apparatus thereby increasing the risk of the hormone exposition significantly.

A further possibility for the granulation is the fluidized bed granulating and drying method in an apparatus wherein a solution or suspension of the active agent is sprayed onto the powder mass. In this case depending on the spray pressure the spraying procedure provides controlled effect on the dispersion degree of liquid particles. However, the small liquid particles have a risk to dry in the air and to leave the operating zone with the fluid airflow before they contact with the powder. Another risk for the liquid particles is to stick to the wall of the apparatus. These effects can influence on the homogeneity of the product and can cause increasing active agent loss. Note, that active agents, especially the hormone-containing substances that leave the apparatus by the airflow always imply some healthy risk. As it can be seen the spraying method does not afford the possibility to make products without active agent loss. The smaller the active agent contents of the system, the greater the occurrence of the above disadvantages. In case of hormone containing products this problem appears in higher degree. A possible solution for the above problem is spraying the active agent in the form of a suspension; however, in this case problems of inhomogeneity may occur.

EP 1214076 discloses a method for the preparation of drospirenone compositions wherein due to the relatively high active agent content of the composition (3 mg/tablet) the drospirenone is processed in micronized form without using any kind of solvent. In this technological procedure the distribution of the active agent in the system can be much more inhomogenous than in the case when the active agent is dispersed in molecular dispersed form. Using the active agent in micronized form during the tabletting procedure, another drawback is the sticking, which can make the manufacturing process of the tablet cores uncertain.

In the pharmaceutical industry tablet cores containing hormones are always coated. The coating procedure plays double roles: it decreases dusting of the product during packaging, and facilitates oral administration of the tablets through the gullet.

Our aim was to provide film-coated tablets containing drospirenone active agent wherein the composition and construction of the film-coated tablet according to the invention eliminates disadvantages of the processes described in the technical literature. Further, our aim was to provide a process for applying coat onto the surface of tablet cores. The process according to the invention is safe and easy-to make in industrial scale and applying the drospirenone active agent onto the core may be accomplished with increased safety.

### Brief description of the invention

The present invention relates to a process for the preparation of cores of film-coated tablets containing drospirenone active agent wherein a solution of the active agent in an organic solvent is dripped continuously or periodically onto the surface of the fluid bed without using spray pressure. At the same time the temperature of the product is controlled and the solvent molecules are removed from the wet material by a hot airflow. Further, the present invention relates to film-coated tablets containing drospirenone active agent with improved resistance against the environmental influences, especially against air humidity wherein coat is applied onto the tablet cores produced according to the above procedure. This coat provides improved resistance against the environmental influences, especially against moisture in case of higher air humidity.

### Aspects of the Invention

The present invention relates to the following items (1) to (5) :
(1) Process for the preparation of film-coated tablets containing drospirenone active agent which comprises dissolving drospirenone in ethanol, then dripping continuously or periodically the solution obtained onto the surface of the fluid bed in a fluidizing equipment without spraying pressure while controlling the temperature of the product obtained and removing the solvent molecules from the wet material by a hot airflow, and the cores obtained are film-coated with an organic and/or aqueous solution or dispersion of a film-forming polymer.
(2) A process according to item (1), wherein the film-forming polymer is selected from the group consisting of acrylic acid, methacrylic acid, methoxyethylacrylate, cellulose acetate, ethylene oxide and polyethylene oxide, ethylene vinyl acetate copolymer, methyl cellulose, polyvinyl acetate, polyvinyl alcohol, polyvinyl pyrrolidone, polytetrafluorethylene and polyvinylidene chloride.
(3) A process according to item (2), wherein the film-forming polymer is amino alkyl methacrylate copolymer E.
(4) Film-coated tablet containing drospirenone active agent comprising tablet cores coated with a film-forming polymer layer with improved resistance against atmospheric humidity wherein the film-forming polymer is selected from the group consisting of acrylic acid, methacrylic acid, methoxyethylacrylate, cellulose acetate, ethylene oxide and polyethylene oxide, ethylene vinyl acetate copolymer, methyl cellulose, polyvinyl acetate, polyvinyl alcohol, polyvinyl pyrrolidone, polytetrafluorethylene and polyvinylidene chloride.
(5) Film-coated tablet according to item (4), wherein the film-forming polymer is amino alkyl methacrylate copolymer E.

### Detailed description of the invention

The present invention relates to a process for the preparation of drospirenone active agent containing film-coated tablet cores wherein a solution of the active agent in an organic solvent is dripped continuously or periodically onto the surface of the fluid bed without using spray pressure while controlling temperature of the product the solvent molecules are removed from the wet material by a hot airflow.

According to the essence of invention the above solution is dripped onto the surface of the power bed without dispersing the drops, hereby the drops are not scattered and whole their quantity fall onto the surface of the power bed. Since the drops are directed to fix point(s) on the surface of the fluid bed, the active agent loss described in the technical literature may be practically avoided. Moreover, by suitable choose of the volume ratio of solution of the active agent to the power bed and by suitable choose of the speed of air the wetted substance is mixed properly and the system is wetted uniformly. In this manner a homogenous distribution of the active agent is achieved. In all cases, the average drop size is higher (0,15 cm³) than the average particle size of the dispersed particles obtainable by the spraying method.

For dissolution of such a large amount of active agent a large amount of pharmaceutically and environmentally acceptable solvent is needed. Since the drospirenone is substantially insoluble in water, the suitable solvents fall under the "Class 3". In the preparation process according to the invention the solvent is ethanol. Ethanol is the most advantageous solvent from the environmental and safety point of views.

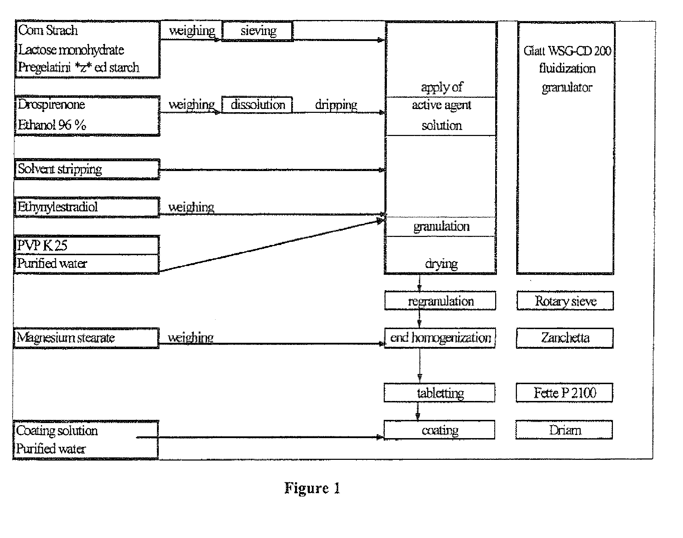

The process according to the invention is showed in the Figure 1. In the flow sheet of Figure 1 the procedure steps are showed. According to the first step in a suitable fluidization equipment a solution of the active agent in ethanol is applied onto the components (corn starch, lactose monohydrate and pregelatinized starch) of the inner phase. Accordingly, the solution of drospirenone in ethanol is poured slowly onto the components of the inner phase without using spray air thereby minimizing the active agent loss. Then the alcohol is stripped from the wet mass. After drying ethynylestradiol is added to the inner phase components pretreated with the solution of drospirenone then the mixture is homogenized and granulated by adding an aqueous solution of polyvinylpyrrolidone. The granules obtained are dried, regranulated and lubricated by adding magnesium stearate and tablets are pressed. Then the tablets obtained are film-coated. Similarly to the spraying method, in case of the process according to the invention the drospirenone active agent obtained at the end of the procedure is in amorphous form in the tablets.

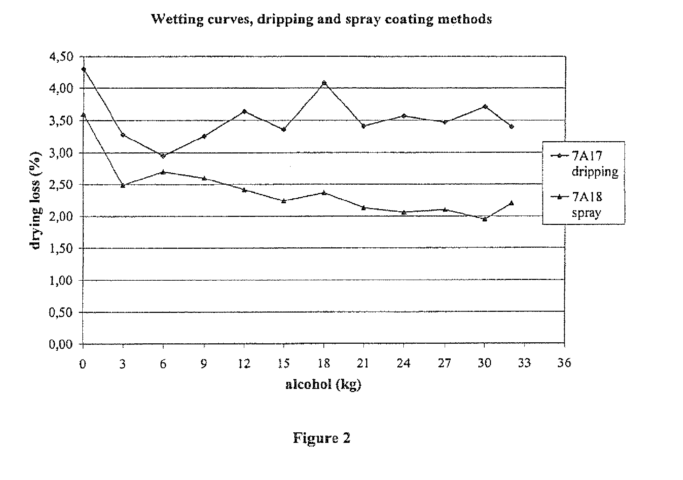

As it can be seen in Figure 2, during the procedure wherein a solution of the active agent in ethanol is applied onto the components of the inner phase, uniform wetting and maintaining wet can be maintained. In the fluid bed the homogenous distribution of the active agent is ensured without using spray air. By omitting spay procedure the active agent loss occurring due to the sticking of the active agent to the wall of the equipment can be minimized.

Further, the present invention relates to film-coated tablets containing drospirenone active agent having improved resistance against the environmental influences, especially against air humidity wherein coat is applied onto the tablet cores produced according to the above process. This coat provides improved resistance against the environmental influences, especially against moisture in case of higher air humidity.

The process according to the invention can be accomplished by using weak hydrophobic polymers that coat the surface of the cores uniformly. Suitable polymers for the present invention include, but are not limited to, acrylic acid, methacrylic acid, methoxyethylacrylate, cellulose acetate, ethylene oxide and polyethylene oxide, ethylene vinyl acetate copolymer, methyl cellulose, polyvinyl acetate, polyvinyl alcohol, polyvinyl pyrrolidone, polytetrafluorethylene, polyvinylidene chloride. Surprisingly, the coat according to the invention does not influence the in vivo biological effect, however, due to its high resistance against humidity, the following in vitro dissolution profile is showed: less than 70% of the active agent is released from the film-coated tablet within 30 seconds when tested at 37 °C temperature in water, above pH=4,5 and stirring at 50 rpm.

The present invention is now explained in detail by the following examples, which are given to illustrate the invention rather than to limit the scope.

### Example 1:

| **Ingredients** | **Amounts** |
|---|---|
| Drospirenone | 3.0 mg |
| Ethynylestradiol | 0.03 mg |
| Lactose monohydrate | 48.67 mg |
| Corn starch | 15.0 mg |
| Modified pregelatinized starch | 10.0 mg |
| PVP | 2.5 mg |
| Magnesium stearate | 0.8 mg |
| Film-coat (Methacrylic acid copolymer) | 2.0 mg |
| **Total:** | **82.0 mg** |

The tablet cores prepared according to the procedure described above are film-coated. For the preparation of the film-coat the following steps are carried out. Under continuous high speed agitation stirring detergent (sodium laurylsulphate) is dispersed in a part of the purified water, and then the stearic acid and polymer components are added to the mixture. In the same time pigment (titan dioxide) is dispersed into the other part of the purified water then the two parts of dispersions obtained are mixed and further stirred. Finally, the coating obtained is sprayed onto the surfaces of tablet cores and dried.

### Example 2:

| **Ingredients** | **Amounts** |
|---|---|
| Drospirenone | 3.0 mg |
| Ethynylestradiol | 0.03 mg |
| Lactose monohydrate | 48.67 mg |
| Corn starch | 15.0 mg |
| Modified pregelatinized starch | 10.0 mg |
| Polyacrylin potassium | 2.5 mg |
| Magnesium stearate | 0.8 mg |
| Film-coat (Methacrylic acid copolymer) | 2.0 mg |
| **Total:** | **82.0 mg** |

The tablet cores prepared according to the procedure described in Example 1 are film-coated. For the preparation of the film-coat the following steps are carried out. Under continuous high speed agitation stirring detergent (sodium laurylsulphate) is dispersed in a part of the purified water, and then the stearic acid and polymer components are added to the mixture. In the same time pigment (titan dioxide) is dispersed into the other part of the purified water then the two parts of dispersions obtained are mixed and further stirred. Finally, the coating obtained is sprayed onto the surfaces of the tablet cores and dried.

### Example 3.

| **Ingredients** | **Amounts** |
|---|---|
| Drospirenone | 3.0 mg |
| Ethynylestradiol | 0.03 mg |
| Lactose monohydrate | 48.67 mg |
| Potato starch | 15.0 mg |
| Modified pregelatinized starch | 10.0 mg |
| Polyacrylin potassium | 2.5 mg |
| Magnesium stearate | 0.8 mg |
| Film-coat (Methacrylic acid copolymer) | 2.0 mg |
| **Total:** | **82.0 mg** |

The tablet cores prepared according to the procedure described above are film-coated. For the preparation of the film-coat the following steps are carried out. Under continuous high speed agitation stirring detergent (sodium laurylsulphate) is dispersed in a part of the purified water, and then the stearic acid and polymer components are added to the mixture. Pigment (titan dioxide) is dispersed into the other part of the purified water then the two parts of dispersions obtained are mixed and further stirred. Finally, the coating obtained is sprayed onto the surfaces of the tablet cores and dried.

## Claims

1. Film-coated tablet containing drospirenone active agent, comprising a tablet core coated with a film-forming polymer layer with improved resistance against atmospheric humidity
wherein the tablet core is obtainable by a process which comprises dissolving drospirenone in ethanol, then dripping continuously or periodically the solution obtained onto the surface of the fluid bed in a fluidizing equipment without spraying pressure while controlling the temperature of the product obtained and removing the solvent molecules from the wet material by a hot airflow;
and wherein the film-forming polymer is selected from the group consisting of acrylic acid, methacrylic acid, methoxyethylacrylate, cellulose acetate, ethylene oxide and polyethylene oxide, ethylene vinyl acetate copolymer, methyl cellulose, polyvinyl acetate, polyvinyl alcohol, polyvinyl pyrrolidone, polytetrafluorethylene and polyvinylidene chloride.

2. Film-coated tablet according to claim 1, wherein the film-forming polymer is amino alkyl methacrylate copolymer E.
